# EUROPÄISCHE PATENTSCHRIFT

(11) **EP 3 185 962 B1**
(45) Veröffentlichungstag und Bekanntmachung des Hinweises auf die Patenterteilung: **26.12.2018**
(21) Anmeldenummer: 15750993.6
(22) Anmeldetag: 12.08.2015
(51) Int. Cl.: A61Q 5/08, A61Q 5/10, A61K 8/22

(54) **MULTITONALE EINSCHRITT-FÄRBUNGEN MIT VERDICKTER VORBEHANDLUNGSLÖSUNG III**
MULTI-TONAL ONE STEP DYEING PROCESS USING A THICKENED PRETREATMENT SOLUTION III
COLORATION MULTITON EN UNE ÉTAPE UTILISANT UN AGENT DE PRÉ-TRAITEMENT ÉPAISSI III

(30) Priorität: 26.08.2014 DE 102014216940
(43) Veröffentlichungstag der Anmeldung: 05.07.2017
(73) Patentinhaber: Henkel AG & Co. KGaA, 40589 Düsseldorf (DE)
(72) Erfinder: NEUBA, Constanze, 41516 Grevenbroich (DE); MÜLLER, Burkhard, 40221 Düsseldorf (DE)
(86) Internationale Anmeldenummer: PCT/EP2015/068558
(87) Internationale Veröffentlichungsnummer: WO 2016/030187

(56) Entgegenhaltungen:
- EP-A1- 1 927 340
- DATABASE CA [Online] CHEMICAL ABSTRACTS SERVICE, COLUMBUS, OHIO, US; 12. September 2013 (2013-09-12), "Hair dyeing method and pretreatment agent for hair dyeing", XP002744945, Database accession no. 159:416500 & WO 2013/129643 A1 (MIZ CO LTD [JP]; KURIMURA MASATO [JP]; MIZTEC COMPANY [JP]) 6. September 2013 (2013-09-06)

## Beschreibung

Gegenstand der vorliegenden Anmeldung ist ein Verfahren zur Behandlung keratinischer Fasern, welches es erlaubt, in einem Färbeschritt das Haar zu colorieren und gleichzeitig eine multitonale Färbung mit helleren ("highlights") Partien (Strähnchen) zu erzeugen. Hierzu wird ein verdicktes Blondierungsmittel in Kombination mit einem oxidativem Färbemittel verwendet.

Im Laufe der Zeit und insbesondere unter Einwirkung von äußeren Einflüssen wie Licht oder atmosphärischen Schadstoffen verliert oder verändert das Haar seine natürliche Farbe und seinen Glanz bzw. Schimmer. Aus diesem Grund finden Haarfärbemittel breite Anwendung, welche entweder im Friseurbereich oder durch die Heimanwendung genutzt werden.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, welche unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, welche als färbende Komponente sogenannte direktziehende Farbstoffe ("Direktzieher") enthalten.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie Wasserstoffperoxid, entfärbt.

Bei der Haarfärbung - insbesondere bei der Haarfärbung durch die Heimanwendung - tritt das Problem auf, dass natürliche Farbnuancierungen vollständig überdeckt werden, so dass multitonale Färbungen schwer zu realisieren sind.

Um dem Haar ein natürlicheres Aussehen zu verleihen, ist es im Stand der Technik bekannt, gefärbte Haare durch die gezielte Anwendung von Oxidationsmitteln partiell zu entfärben. Die Haarpartien ("Strähnchen"), auf welche die Oxidationsmittel aufgetragen werden, bleichen dabei mindestens anteilsweise aus, woraus eine multitonale Haarfarbe resultiert. Die Applikation des Oxidationsmittels erfolgt dabei mit einer Bürste oder einem Pinsel, wobei die nicht zu behandelnden Haare gegebenenfalls mit Aluminiumfolie oder einer sogenannten "Strähnchenhaube" vor der Entfärbung geschützt werden. Diese Applikationsart löst zwar das Problem der möglichst natürlichen Färbung von Haaren, jedoch ist ein zeitaufwendiger zweiter Entfärbeschritt erforderlich, welcher sich an die erste Färbung anschließt. Insbesondere bei der Heimanwendung müsste daher zunächst das gesamte Haar coloriert werden, bevor die Anwenderin "highlights" setzen kann. Von vielen Verbraucherinnen wird dies als zu zeitaufwendig und auch als frustrierend empfunden, da der wesentliche farbverändernde Schritt anfangs erfolgt und in einem zweiten Schritt lediglich "korrigiert" wird.

WO 2013/129643 A1 offenbart ein oxidatives Haarfärbeverfahren, bei welchem ein oxidatives Vorbehandlungsmittel enthaltend Wasserstoffperoxid und Wasserstoff eingesetzt wird.

Der vorliegenden Erfindung lag die Aufgabe zugrunde, ein Verfahren bereitzustellen, welches multitonale Färbungen in einem Färbeschritt ermöglicht. Dabei sollte die Färbung des Haares mit der Erzeugung von "highlights" einhergehen, damit nach dem Ausspülen des Färbemittels direkt ein Ergebnis sichtbar ist.

Es wurde nun gefunden, dass eine partielle Vorbehandlung von Faserarealen bzw. Strähnchen dazu führt, dass diese Areal bzw. Strähnchen später weniger intensiv angefärbt werden. Durch die Vorpenetration bzw. Vorbehandlung einzelner Faserareale bzw. Strähnchen färbt das unmittelbar darauffolgend angewendete Färbemittel das Haar multitonal und es wird direkt nach dem Färbeschritt ein natürliches Färbeergebnis mit "highlights" erhalten.

Ein Gegenstand der vorliegenden Erfindung ist daher ein Verfahren zum oxidativen Färben von keratinischen Fasern gemäß Anspruch 1.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn das erfindungsgemäße Verfahren zur Färbung von menschlichen Haaren verwendet wird.

Weiterhin sind unter dem Begriff "Verdickungsmittel" im Rahmen der vorliegenden Erfindung Verbindungen zu verstehen, welche Flüssigkeiten, insbesondere Wasser, binden können und die Viskosität dieser Flüssigkeiten erhöhen. Hierzu gehören im Rahmen der vorliegenden Erfindung auch Gelbildner, welche in der Lage sind, Flüssigkeiten zu Zusammensetzungen mit gelartiger Konsistenz bzw. zu Gelen zu verdicken. Unter gelartigen kosmetischen Mitteln bzw. Gelen werden erfindungsgemäß formbeständige, leicht deformierbare disperse Systeme aus mindestens zwei Komponenten, dem Gelbildner (meist ein fester, kolloidzerteilter Stoff mit langen oder stark verzweigten Verbindungen) und einer Flüssigkeit (meist Wasser) als Dispersionsmittel, verstanden. Der Gelbildner bildet in der Flüssigkeit ein räumliches Netzwerk aus, wobei die einzelnen gelbildenden Verbindungen durch Haupt- und/oder Nebenvalenzen an verschiedenen räumlichen Punkten untereinander aneinanderhaften. Zudem sind unter dem Begriff der "Fettsäuren", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff der "Fettalkohole" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Erfindungsgemäß bevorzugt werden die Verfahrensschritte a) bis e) in der zuvor angeführten Reihenfolge mit einem Zeitabstand zwischen den einzelnen Verfahrensschritten von jeweils 0 bis 60 Minuten, vorzugsweise von jeweils 0 bis 40 Minuten, insbesondere von jeweils 0 bis 30 Minuten, durchgeführt.

Im ersten Verfahrensschritt (Verfahrensschritt a)) des erfindungsgemäßen Verfahrens wird ein kosmetisches Mittel (M1) auf die Fasern appliziert. Dieses kosmetische Mittel (M1), welches nachfolgend auch als Vorbehandlungsmittel oder als Vorpenetrationsmittel bezeichnet wird, wird über einen Zeitraum von 1 Minute bis 60 Minuten auf den keratinischen Fasern belassen (Verfahrensschritt b) des erfindungsgemäßen Verfahrens).

Erfindungsgemäß bevorzugt sind jedoch eher kürzere Einwirkzeiten des Vorbehandlungsmittels. Besonders bevorzugte erfindungsgemäße Verfahren sind dadurch gekennzeichnet, dass das kosmetische Mittel (M1) in Verfahrensschritt b) für einen Zeitraum von 2 bis 60 Minuten, vorzugsweise von 3 bis 45 Minuten, insbesondere von 5 bis 30 Minuten, auf den keratinischen Fasern Einwirken gelassen wird. Die Vorbehandlung von keratinischen Fasern mit dem kosmetischen Mittel (M1) führt dazu, dass an diesen Stellen bereits Inhaltsstoffe des Vorbehandlungsmittels (M1) an den keratinischen Fasern haften bzw. in die keratinischen Fasern eingedrungen sind, so dass das Färbeergebnis bei anschließendem Auftragen des kosmetischen Mittels (M2) an diesen Stellen aufgehellt wird. Auf diese Weise kann in einem Färbeschritt das Haar coloriert und gleichzeitig eine multitonale Färbung mit helleren ("highlights") Partien (Strähnchen) erzeugt werden.

Es hat sich gezeigt, dass eine Vorbehandlung bei leicht erhöhten Temperaturen die multitonalen Effekte noch lebendiger wirken lässt. Erfindungsgemäß bevorzugte Verfahren sind dadurch gekennzeichnet, dass das kosmetische Mittel (M1) in Verfahrensschritt b) bei einer Temperatur von 20 °C bis 120 °C, insbesondere 30 °C bis 120 °C, Einwirken gelassen wird. Temperaturen von 20 °C bis 120 °C, bevorzugt von 30 °C bis 120 °C können beispielsweise unter Verwendung eines Heißluftföns oder einer Trockenhaube erreicht werden.

Um multitonale Färbungen zu erzeugen, werden nur einzelne Strähnen mit dem kosmetischen Mittel (M1) beaufschlagt. Alternativ kann die auf einzelne Strähnchen aufgetragene Konzentration des kosmetischen Mittels (M1) variiert werden. Es ist auch möglich, das kosmetische Mittel (M1) zunächst gleichmäßig auf die gesamten keratinischen Fasern zu applizieren und anschließend einzelne Bereiche bzw. Strähnchen erneut mit dem kosmetischen Mittel (M1) zu behandeln. Auch eine mehrmalige Behandlung einzelner Bereiche/Strähnen mit dem kosmetischen Mittel (M1) ist erfindungsgemäß möglich.

Das kosmetische Mittel (M1) wird in Verfahrensschritt a) nur auf einzelne Strähnchen appliziert Unter dem Begriff "Strähnchen" wird erfindungsgemäß ein von der Gesamtheit an keratinischen Fasern abgetrennter Teil verstanden, welcher aus mindestens 2, bevorzugt mindestens 50, insbesondere mindestens 100, keratinischen Fasern besteht.

Im Anschluss an die Einwirkzeit des Vorbehandlungsmittels werden die keratinischen Fasern nicht ausgespült oder abfrottiert. Vielmehr wird in Verfahrensschritt c) des erfindungsgemäßen Verfahrens ein kosmetisches Mittel (M2) auf die noch mit dem kosmetischen Mittel (M1) beaufschlagten keratinischen Fasern appliziert. Die durch Applikation des kosmetischen Mittels (M2) auf den keratinischen Fasern entstandene Mischung aus den kosmetischen Mitteln (M1) und (M2) wird in Verfahrensschritt d) des erfindungsgemäßen Verfahrens für einen Zeitraum von 1 bis 70 Minuten Einwirken gelassen.

Erfindungsgemäß sind jedoch eher kürzere Einwirkzeiten der kosmetischen Mittel (M1) und (M2) in Verfahrensschritt d) bevorzugt. Besonders bevorzugte erfindungsgemäße Verfahren sind dadurch gekennzeichnet, dass die kosmetischen Mittel (M1) und (M2) in Verfahrensschritt d) für einen Zeitraum von 1 bis 60 Minuten, vorzugsweise von 5 bis 50 Minuten, insbesondere von 10 bis 45 Minuten, Einwirken gelassen werden.

Da das kosmetische Mittel (M1) in Verfahrensschritt b) des erfindungsgemäßen Verfahrens bereits eine gewisse Zeit auf den keratinischen Fasern belassen wurde, haben diese keratinischen Fasern zu den Inhaltsstoffen des kosmetischen Mittels (M1) einen längeren Kontakt als zu denen des kosmetischen Mittels (M2). Wenn das kosmetische Mittel (M1) nur auf einzelne Strähnchen bzw. in einzelnen Bereichen abgewendet wurde, konnten die Inhaltsstoffe des kosmetischen Mittels (M1) in diesen Bereichen intensiver einwirken und somit die Wirkung der Inhaltsstoffe des kosmetischen Mittels (M2) in diesen Bereichen abschwächen, wodurch eine hellere Färbung dieser Bereiche erzielt wird.

Nach dem Ausspülen der kosmetischen Mittel (M1) und (M2) in Verfahrensschritt e) des erfindungsgemäßen Verfahrens wird, ohne einen weiteren Schritt durchführen zu müssen, unmittelbar ein multitonales Farbergebnis erhalten.

Bei dem kosmetischen Mittel (M1) bzw. dem Vorbehandlungsmittel handelt es sich um ein Bleichmittel, welches mindestens ein Oxidationsmittel (M1-1) enthält. Als Oxidationsmittel kommen Persulfate, Peroxodisulfate, Chlorite, Hypochlorite und insbesondere Wasserstoffperoxid und/oder eines seiner festen Anlagerungsprodukte an organische oder anorganische Verbindungen in Frage.

Erfindungsgemäß bevorzugte Verfahren sind daher dadurch gekennzeichnet, dass das mindestens eine Oxidationsmittel (M1-1) ausgewählt ist aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat und deren Mischungen.

Bei besonders bevorzugten erfindungsgemäßen Verfahren ist das mindestens eine Oxidationsmittel (M1-1) ausgewählt ist aus der Gruppe von Wasserstoffperoxid sowie dessen feste Anlagerungsprodukte an Harnstoff, Melamin, Polyvinylpyrrolidon und Natriumborat, bevorzugt Wasserstoffperoxid.

Um eine ausreichende Aufhellung der mit dem Vorbehandlungsmittel behandelten Haare zu gewährleisten, ist es im Rahmen der vorliegenden Erfindung bevorzugt, wenn das kosmetische Mittel (M1) das mindestens eine Oxidationsmittel (M1-1) in einer Gesamtmenge von 1,0 bis 8,0 Gew.-%, vorzugsweise von 1,5 bis 7,5 Gew.-%, bevorzugt von 2,0 bis 7,0 Gew.-%, weiter bevorzugt von 2,5 bis 7,0 Gew.-%, insbesondere von 3,0 bis 7,0 Gew.-%, bezogen auf das Gesamtgewicht des kosmetischen Mittels (M1), enthält. Falls als Oxidationsmittel Wasserstoffperoxid sowie dessen feste Anlagerungsprodukte eingesetzt werden, berechnet sich die vorstehend genannte Gesamtmenge auf 100%iges H₂O₂.

In einer weiteren bevorzugten Ausführungsform ist das kosmetische Mittel (M1) ein Mittel zum Aufhellen bzw. Bleichen keratinischer Fasern, welches als Oxidationsmittel (M1-1) Wasserstoffperoxid oder dessen feste Anlagerungsprodukte an Harnstoff, Melamin, Polyvinylpyrrolidon und Natriumborat, bevorzugt Wasserstoffperoxid in einer Gesamtmenge von 1,0 bis 12 Gew.-%, vorzugsweise von 1,5 bis 12 Gew.-%, bevorzugt von 2,0 bis 12 Gew.-%, weiter bevorzugt von 3,0 bis 12 Gew.-%, insbesondere von 3,5 bis 12,0 Gew.-%, bezogen auf das Gesamtgewicht des kosmetischen Mittels (M1), enthält. Die vorstehend genannte Gesamtmenge an Wasserstoffperoxid ist hierbei ebenfalls auf 100%iges H₂O₂ bezogen.

Zur Erzielung einer verstärkten Aufhell- und Bleichwirkung kann das kosmetische Mittel (M1) weiterhin mindestens ein Peroxo-Salz enthalten. Geeignete Peroxo-Salze sind anorganische Peroxoverbindungen, bevorzugt ausgewählt aus der Gruppe von Ammoniumperoxodisulfat, Alkalimetallperoxodisulfaten, Ammoniumperoxomonosulfat, Alkalimetallperoxomonosulfaten, Alkalimetallperoxodiphosphaten, Erdalkalimetallperoxiden sowie deren Mischungen. Besonders bevorzugt sind Peroxodisulfate, insbesondere Ammoniumperoxodisulfat, Kaliumperoxodisulfat und Natriumperoxodisulfat. Die zuvor genannten Peroxosalze sind in einer Gesamtmenge von 0,5 bis 20 Gew.-%, vorzugsweise 1 bis 12,5 Gew.-%, bevorzugt 2,5 bis 10 Gew.-%, insbesondere 3 bis 6 Gew.-%, bezogen auf das Gesamtgewicht des kosmetischen Mittels (M1), enthalten.

Die im Rahmen des erfindungsgemäßen Verfahrens verwendeten Vorbehandlungsmittel (M1) weisen einen basischen pH-Wert, von pH 7 bis pH 14 auf. Diese pH-Werte sind erforderlich, um eine Öffnung der äußeren Schuppenschicht (Cuticula) zu gewährleisten und eine Penetration der Oxidationsfarbstoffvorprodukte in das Haar zu ermöglichen.

Erfindungsgemäß bevorzugte Verfahren sind daher dadurch gekennzeichnet, dass das kosmetische Mittel (M1) einen pH-Wert (M1-2) von pH 7,5 bis pH 13, vorzugsweise von pH 8,0 bis pH 12,5, bevorzugt von pH 8,0 bis pH 12, weiter bevorzugt von pH 8,0 bis pH 11,5, insbesondere von pH 8,0 bis pH 11, aufweist. Die Einstellung dieser pH-Werte kann bevorzugt unter Verwendung der von nachfolgend angeführten Alkalisierungsmitteln erfolgen.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, welcher mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol sowie deren Mischungen. Ein insbesondere bevorzugtes Alkanolamin ist Monoethanolamin. Geeignete basische Aminsäuren sind Lysin, Arginin und Ornithin. Erfindungsgemäße, anorganische Alkalisierungsmittel sind bevorzugt ausgewählt aus der Gruppe von Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Erfindungsgemäß besonders bevorzugte Verfahren sind dadurch gekennzeichnet, dass das kosmetische Mittel (M1) ein oder mehrere Alkalisierungsmittel aus der Gruppe von Natriumhydroxid, Kaliumhydroxid, Ammoniak, Monoethanolamin und 2-Amino-2-methylpropanol in einer Gesamtmenge von 0,05 bis 8,0 Gew.-%, vorzugsweise von 0,1 bis 6,0 Gew.-%, insbesondere von 0,5 bis 5,0 Gew.-%, bezogen auf das Gesamtgewicht des kosmetischen Mittels (M1), enthält.

Um das natürliche und multitonale Färbeergebnis am Ende des erfindungsgemäßen Verfahrens besonders deutlich und überraschend hervortreten zu lassen, ist das Vorbehandlungsmittel (M1) für sich alleine vorzugsweise nicht in der Lage, als separates Färbemittel eingesetzt zu werden. Aus diesem Grund enthalten die kosmetischen Mittel (M1) keine Verbindung aus der Gruppe der Oxidationsfarbstoffvorprodukte vom Entwicklertyp und Kupplertyp, der direktziehenden Farbstoffe sowie deren Mischungen (M1-3).

Erfindungsgemäß bevorzugte Verfahren sind daher dadurch gekennzeichnet, dass das in Verfahrensschritt a) eingesetzte kosmetische Mittel (M1) 0 Gew.-%, bezogen auf das Gesamtgewicht des kosmetischen Mittels (M1), einer Verbindung aus der Gruppe der Oxidationsfarbstoffvorprodukte vom Entwicklertyp und Kupplertyp, der direktziehenden Farbstoffe sowie deren Mischungen enthält. Oxidationsfarbstoffvorprodukte vom Entwicklertyp und vom Kupplertyp bilden im Rahmen der oxidativen Färbung ausschließlich unter Verwendung eines Oxidationsmittels durch Ausbildung kovalenter Bindungen untereinander die gewünschte Färbung aus. Dahingegen handelt es sich bei direktziehenden Farbstoffen um Farbstoffe, welche direkt auf das Haar aufziehen und keinen oxidativen Prozess, d.h. keine Verwendung eines Oxidationsmittels, zur Ausbildung der Farbe benötigen.

Um das Vorpenetrationsmittel (M1) sauber und lokal begrenzt applizieren zu können, hat sich eine gelartige Konsistenz des Mittels als vorteilhaft erwiesen. Die gelartigen Vorbehandlungsmittel (M1) gewährleisten zum einen eine gute und gleichmäßige Verteilbarkeit auf den keratinischen Fasern und führen nicht zu einem Verlaufen oder Ausfließen während der Einwirkzeit in Verfahrensschritt b). Auf diese Weise ist die Applikation und Einwirkung des Vorbehandlungsmittels (M1) auf begrenzte Strähnchen bzw. Bereiche möglich, so dass ein hervorragendes multitonales Färbeergebnis ohne Verwischen der Strähnchen infolge eines Verlaufens des Vorbehandlungsmittels (M1) resultiert.

Das kosmetische Mittel (M1) weist eine dynamische Viskosität von 10.000 bis 50.000 mPa*s auf, gemessen mit Brookfield RDV II+, Spindel Nr. 4, 4 rpm, 20 °C.

Zur Einstellung der zuvor angeführten Viskosität werden insbesondere Verdickungsmittel, Fettalkohole, mit Alkalisierungsmitteln verseifte Fettsäuren sowie deren Mischungen eingesetzt. Bevorzugte Verfahren im Rahmen der vorliegenden Erfindung sind daher dadurch gekennzeichnet, dass das kosmetische Mittel (M1) zusätzlich mindestens eine viskositätserhöhende Verbindung aus der Gruppe der Verdickungsmittel, der Fettalkohole, der mit Alkalisierungsmittel verseiften Fettsäuren sowie deren Mischungen enthält. Unter dem Begriff "viskositätserhöhende Verbindungen" werden Verbindungen verstanden, deren Zugabe zu dem kosmetischen Mittel (M1) zu einer Erhöhung der Viskosität dieses Mittels führt. Weiterhin werden unter dem Begriff "mit Alkalisierungsmitteln verseifte Fettsäuren" werden salzartige Verbindungen verstanden, in welchen die Fettsäure als Anion und das Alkalisierungsmittel als Kation vorliegen.

Wenn zur Einstellung der zuvor angeführten Viskosität Verdickungsmittel eingesetzt werden, sind diese bevorzugt ausgewählt aus der Gruppe von verdickenden Polysacchariden, verdickenden synthetischen Polymeren, verdickenden anorganischen Verbindungen sowie deren Mischungen.

In diesem Zusammenhang kann es vorgesehen sein, dass das verdickende Polysaccharid ausgewählt ist aus der Gruppe von Xanthan, Cellulosen, Cellulosederivaten, Curdlan, Alginen, Alginaten, Glucanen, Pullulanen, Amylosen, Traganth, Karaya-Gummi, Ghatti-Gummi, Agar, Carrageenan, Chitin, Chitosan, Gummi arabicum, Gellan, Guar Gum, Johannisbrotkernmehl sowie deren Mischungen, bevorzugt Xanthan, Cellulosen, Cellulosederivaten sowie deren Mischungen.

Im Rahmen dieser Ausführungsform kann es weiterhin vorgesehen sein, dass das verdickende synthetische Polymer ausgewählt ist aus der Gruppe von vernetzten Homo- oder Copolymeren der Acrylsäure, der Methacrylsäure sowie deren Salzen und Alkylestern, Homo- oder Copolymere von Acrylsäureamiden und/oder Methacrylsäureamiden, Copolymere aus Acrylsäure und Acrylsäureamiden sowie deren Mischungen, bevorzugt vernetzten Homo- oder Copolymeren der Acylsäure, der Methacrylsäure sowie deren Salzen und Alkylestern, vernetzten Copolymeren der ethoxylierten Alkylester der Methacrylsäure und der sulfonierten Acrylsäureamide sowie deren Salzen und vernetzten Copolymeren der Methacrylsäure, der Acrylsäureamide und der sulfonierten Acrylsäureamide sowie deren Salzen. Derartige Polymere sind beispielsweise das unter der INCI-Bezeichnung bekannte vernetzte Copolymer Ammonium Acryloyldimethyltaurate / Beheneth-25 methacrylate Crosspolymer (Handelsbezeichnung: Aristoflex HMB; Clariant), das unter der INCI-Bezeichnung bekannte vernetzte Copolymer Acrylates/C10-30 Alkyl Acrylate Crosspolymer (Handelsbezeichnung: Carbopol; Lubrizol) sowie das unter der INCI-Bezeichnung bekannte vernetzte Copolymer Polyacrylate Crosspolymer-11 (Handelsbezeichnung: Aristoflex Velvet; Clariant).

Darüber hinaus kann es im Rahmen dieser Ausführungsform auch vorgesehen sein, dass die verdickende anionische Verbindung ausgewählt ist aus der Gruppe von Elektrolyten, insbesondere Natriumchlorid und Kaliumchlorid, Schichtsilicaten, Magnesium-Aluminium-Silicate, gegebenenfalls modifizierte Bentonite, insbesondere gegebenenfalls modifizierte Smektite sowie deren Mischungen.

Besonders bevorzugt eingesetzte Verdickungsmittel sind ausgewählt ist aus der Gruppe von Cellulose, Cellulosederivaten, Xanthan, vernetzten Homo- oder Copolymeren der Acrylsäure, der Methacrylsäure und deren Salzen, vernetzten Copolymeren der ethoxylierten Alkylester der Methacrylsäure und der sulfonierten Acrylsäureamide sowie deren Salzen, vernetzten Copolymeren der Methacrylsäure, der Acrylsäureamide und der sulfonierten Acrylsäureamide sowie deren Salzen und Mischungen dieser Verdickungsmittel

Neben den zuvor beschriebenen Verdickungsmitteln können jedoch auch Fettalkohole, ausgewählt aus der Gruppe von Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brasidylalkohol sowie deren technische Mischungen, welche bei der Hochdruckhydrierung von technischen Methylestern auf Basis von Fetten und Ölen oder Aldehyden aus der Roelenschen Oxosynthese sowie als Monomerfraktion bei der Dimerisierung von ungesättigten Fettalkoholen anfallen, eingesetzt werden. Im Rahmen der vorliegenden Erfindung besonders bevorzugte Fettalkohole sind ausgewählt aus der Gruppe von Laurylalkohol, Cetylalkohol, Cetearylalkohol, Stearylalkohol, Palmoleylalkohol, Isostearylalkohol sowie deren Mischungen.

Darüber hinaus eignet sich im Rahmen der vorliegenden Erfindung auch der Einsatz von Fettsäuren, ausgewählt aus der Gruppe von Ölsäure, Linolensäure, Palmitinsäure, Erucasäure, 2-Hexyldodecansäure, 2-Octyldodecansäure, Isostearinsäure sowie deren Mischungen. Diese Fettsäuren werden von den zuvor angeführten Alkalisierungsmitteln verseift, d.h. es bildet sich ein Addukt zwischen Fettsäureanion und Alkalisierungsmittel-Kation aus. Infolge der Verseifung erfolgt eine Verdickung der erfindungsgemäßen kosmetischen Mittel (M1) zu der zuvor genannten, gewünschten Viskosität.

Im Rahmen des erfindungsgemäßen Verfahrens kann es zudem besonders bevorzugt sein, wenn zwei voneinander verschiedene der zuvor genannten viskositätserhöhenden Verbindungen eingesetzt werden.

Um die zuvor angeführte Viskosität einzustellen, ist es im Rahmen der vorliegenden Erfindung bevorzugt, wenn das kosmetische Mittel (M1) das zusätzlich mindestens eine viskositätserhöhende Verbindung in einer Gesamtmenge von 0,1 bis 10 Gew.-%, vorzugsweise von 0,3 bis 7,5 Gew.-%, bevorzugt von 0,5 bis 7,0 Gew.-%, weiter bevorzugt von 0,7 bis 6,5 Gew.-%, insbesondere von 0,8 bis 6,0 Gew.-%, bezogen auf das Gesamtgewicht des kosmetischen Mittels (M1), enthält. Die zuvor genannten Mengen der viskositätserhöhenden Verbindung gewährleisten eine ausreichende Verdickung, so dass ein Verlaufen des Vorbehandlungsmittels (M1) während der Einwirkzeit in Verfahrensschritt b) und ein hierdurch verursachtes Verwischen des multitonalen Färbeergebnisses verhindert wird. Weiterhin stellen diese Mengen an viskositätserhöhender Verbindung eine gute und gleichmäßige Verteilbarkeit der Vorbehandlungsmittel (M1) auf den keratinischen Fasern sicher.

Zusätzlich zu den Verdickungsmittel(n) kann das im erfindungsgemäßen Verfahren eingesetzte kosmetische Mittel (M1) weitere Inhaltsstoffe enthalten. Erfindungsgemäß bevorzugt enthält das kosmetische Mittel (M1) zusätzlich mindestens eine weitere Verbindung, ausgewählt aus der Gruppe von (i) Tensiden; (ii) Glykolen; (iii) Siliconen; (iv) Komplexbildnern; (v) kationischen Polymeren sowie (vi) deren Mischungen.

Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Im Rahmen der vorliegenden Erfindung einsetzbare Tenside sind ausgewählt aus der Gruppe von nichtionischen Tensiden, anionischen Tensiden, amphoteren Tensiden, zwitterionischen Tensiden, kationischen Tensiden sowie deren Mischungen.

Erfindungsgemäß besonderes bevorzugt werden in dem erfindungsgemäßen Verfahren kosmetische Mittel (M1) eingesetzt, welche zusätzlich mindestens ein anionisches Tensid aus der Gruppe der Alkylsulfate, Alkylethersulfate mit 1 bis 20, insbesondere 1 bis 10, Oxyethylengruppen, Ethercarbonsäuren mit 10 bis 20 Kohlenstoffatomen in der Alkylgruppe und bis zu 16 Glykolethergruppen im Molekül sowie deren Mischungen enthalten.

Besonders bevorzugte erfindungsgemäße Verfahren sind daher dadurch gekennzeichnet, dass die kosmetischen Mittel (M1) zusätzlich mindestens ein anionisches Tensid aus der Gruppe von Alkylethersulfaten mit 1 bis 20, insbesondere 1 bis 10 Oxyethylengruppen in einer Gesamtmenge von 0,1 bis 10 Gew.%, vorzugsweise von 0,5 bis 8,0 Gew.%, insbesondere von 0,8 bis 5,0 Gew.%, bezogen auf das Gesamtgewicht des kosmetischen Mittels (M1), enthalten.

Weiterhin werden in dem erfindungsgemäßen Verfahren besonders bevorzugt kosmetische Mittel (M1) eingesetzt, welche zusätzlich mindestens ein kationisches Tensid aus der Gruppe der quartären Ammoniumverbindungen, der Esterquats, der Amidoamine sowie deren Mischungen enthalten.

Besonders bevorzugte erfindungsgemäße Verfahren sind daher dadurch gekennzeichnet, dass die kosmetischen Mittel (M1) zusätzlich mindestens ein kationisches Tensid aus der Gruppe von Alkyltrimethylammoniumchloriden, Dialkyldimethylammoniumchloriden und Trialkylmethylammoniumchloriden in einer Gesamtmenge von 0,01 bis 5,0 Gew.%, vorzugsweise von 0,05 bis 3,0 Gew.%, insbesondere von 0,1 bis 1,5 Gew.%, bezogen auf das Gesamtgewicht des kosmetischen Mittels (M1), enthalten.

Darüber hinaus werden in dem erfindungsgemäßen Verfahren besonders bevorzugt kosmetische Mittel (M1) eingesetzt, welche zusätzlich mindestens ein nichtionisches Tensid aus der Gruppe der Fettsäuremonoethanolamide, der Anlagerungsprodukte von 5 bis 60 Mol, insbesondere 20 bis 40 Mol, Ethylenoxid an Rizinusöl sowie gehärtetes Rizinusöl, der ethoxylierten Glycerylcarbonsäureester mit einem Ethoxylierungsgrad von 2 bis 20, der Alkyloligoglucoside mit 8 bis 16 Kohlenstoffatomen in der Alkylgruppe sowie deren Mischungen enthalten.

Besonders bevorzugte erfindungsgemäße Verfahren sind daher dadurch gekennzeichnet, dass die kosmetischen Mittel (M1) zusätzlich mindestens ein nichtionisches Tensid aus der Gruppe von Fettsäuremonoethanolamiden, insbesondere N-(2-Hydroxyethyl)-kokosfettsäureamid, in einer Gesamtmenge von 0,1 bis 6,0 Gew.%, vorzugsweise von 0,5 bis 4,5 Gew.%, insbesondere von 1,0 bis 3,5 Gew.%, bezogen auf das Gesamtgewicht des kosmetischen Mittels (M1), enthalten.

Darüber hinaus ist es ebenfalls möglich, dass die Vorbehandlungsmittel (M1) zusätzlich mindestens ein zwitterionisches und/oder amphoteres Tensid enthalten. Bevorzugte zwitterionische Tenside sind Betaine, N-Alkyl-N,N-dimethylammonium-glycinate, N-Acyl-aminopropyl-N,N-di-methylammoniumglycinate, und 2-Alkyl-3-carboxymethyl-3-hydroxyethyl-imidazoline. Ein besonders bevorzugtes zwitterionisches Tensid ist unter der INCI-Bezeichnung Cocamidopropyl Betaine bekannt. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, Kokosacylaminoethylaminopropionat und C₁₂-C₁₈-Acyl-sarcosin. Die zwitterionischen und/oder amphoteren Tenside werden einer Gesamtmenge von 0,1 bis 45 Gew.-%, vorzugsweise von 1 bis 30 Gew.-%, insbesondere von 1 bis 15 Gew.-%, bezogen auf das Gesamtgewicht des kosmetischen Mittels (M1), eingesetzt.

Die Vorbehandlungsmittel (M1) können zusätzlich mindestens eine Verbindung aus der Gruppe der Glykole enthalten. Unter dem Begriff "Glykole" werden erfindungsgemäß Verbindungen verstanden, welche 2 Hydroxylgruppen aufweisen.

Erfindungsgemäß geeignete Glykole sind ausgewählt aus der Gruppe von Ethylenglykol, Propylenglykol (1,2-Propandiol), Ethylenglykolmonomethylether, Trimethylenglykol, Triethylenglykol, Polyethylenglykol, Neopentylglykol sowie deren Mischungen.

Besonders bevorzugte erfindungsgemäße Verfahren sind dadurch gekennzeichnet, dass die kosmetischen Mittel (M1) zusätzlich mindestens ein Glykol aus der Gruppe von Ethylenglykol, Propylenglykol (1,2-Propandiol), Polyethylengykol sowie deren Mischungen in einer Gesamtmenge von 0,1 bis 10 Gew.%, vorzugsweise von 0,5 bis 5 Gew.%, insbesondere von 0,8 bis 3 Gew.%, bezogen auf das Gesamtgewicht des kosmetischen Mittels (M1), enthalten.

Die in Verfahrensschritt a) eingesetzten kosmetischen Mittel (M1) können weiterhin mindestens ein Silikon enthalten. Unter dem Begriff der Silikone versteht der Fachmann verschiedene Strukturen siliciumorganischer Verbindungen.

Bevorzugt können die Silikone ausgewählt sein aus mindestens einem Vertreter der Gruppe siliciumorganischer Verbindungen, welche gebildet wird aus
(i) Polyalkylsiloxanen, Polyarylsiloxanen, Polyalkylarylsiloxanen, welche flüchtig oder nicht flüchtig, geradkettig, verzweigt oder zyklisch, vernetzt oder nicht vernetzt sind;
(ii) Polysiloxanen, welche in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)-Polyoxyalkylen(B)-Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, welche eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Mischungen.

Besonders bevorzugte erfindungsgemäße Verfahren sich dadurch gekennzeichnet, dass die kosmetischen Mittel (M1) zusätzlich mindestens ein Silikon ausgewählt aus der Gruppe von Dimethiconen, Amodimethiconen, Dimethiconolen sowie deren Mischungen in einer Gesamtmenge von 0,001 bis 2,0 Gew.-%, bevorzugt von 0,005 bis 1,5 Gew.-%, insbesondere von 0,01 bis 0,5 Gew.-%, bezogen auf das Gesamtgewicht des kosmetischen Mittels (M1), enthalten.

Es hat sich als vorteilhaft erwiesen, wenn die erfindungsgemäß eingesetzten kosmetischen Mittel (M1) zur Stabilisierung des Oxidationsmittels, insbesondere des Wasserstoffperoxids, zusätzlich mindestens einen Stabilisator oder Komplexbildner enthalten.

Besonders bevorzugte erfindungsgemäße Verfahren sind daher dadurch gekennzeichnet, dass die kosmetischen Mittel (M1) Stabilisatoren oder Komplexbildner aus der Gruppe von EDTA und EDDS, Phosphonaten, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) und deren Natriumsalze sowie deren Mischungen in einer Gesamtmenge 0,001 bis 5,0 Gew.-%, vorzugsweise von 0,005 bis 2,5 Gew.-%, bevorzugt von 0,01 bis 1,5 Gew.-% insbesondere von 0,05 bis 1,0 Gew.-%, bezogen auf das Gesamtgewicht des kosmetischen Mittels (M1), enthalten.

Im Rahmen der vorliegenden Erfindung hat es sich weiterhin als vorteilhaft erwiesen, wenn die kosmetischen Mittel (M1) mindestens ein kationisches Polymer enthalten. Unter erfindungsgemäß geeigneten kationischen Polymeren sind Polymere zu verstehen, welche in der Haupt- und/oder Seitenkette "temporär kationische" oder "permanent kationische" Gruppen aufweisen. Als "permanent kationisch" werden erfindungsgemäß solche Polymere bezeichnet, welche unabhängig vom pH-Wert des Mittels eine kationische Gruppe aufweisen. Dies sind in der Regel Polymere, welche ein quartäres Stickstoffatom, beispielsweise in Form einer Ammoniumgruppe, enthalten.

Bevorzugte kationische Gruppen enthalten quartäre Ammoniumgruppen. Insbesondere solche Homo- und Copolymere, bei welchen die quartäre Ammoniumgruppe über eine C₁₋₄-Kohienwasserstoffgruppe an eine aus Acrylsäure, Methacrylsäure oder deren Derivaten aufgebaute Polymerhauptkette gebunden sind, haben sich als besonders geeignet erwiesen. Derartige Homo- und Copolymere sind beispielsweise unter den Handelsbezeichnungen Salcare® SC 95, Salcare® SC 96 und Salcare® SC 92 im Handel erhältlich.

Gleichfalls als kationische Polymere eingesetzt werden können die unter den Bezeichnungen Polyquaternium-2, Polyquaternium-8, Polyquaternium-17, Polyquaternium-24 (Handelsprodukt z. B. Quatrisoft® LM 200), Polyquaternium-27 sowie Polyquaternium-87 bekannten Polymere.

Besonders bevorzugte erfindungsgemäße Verfahren sind dadurch gekennzeichnet, dass die kosmetischen Mittel (M1) mindestens ein kationisches Polymer aus der Gruppe von Polyquaternium-2, Polyquaternium-8, Polyquaternium-17, Polyquaternium-24, Polyquaternium-27 sowie Polyquaternium-87 in einer Gesamtmenge von 0,1 bis 5,0 Gew.-%, vorzugsweise von 0,2 bis 3,0 Gew.-%, insbesondere von 0,5 bis 2,0 Gew.-%, bezogen auf das Gesamtgewicht des kosmetischen Mittels (M1), enthalten.

Um eine vorzeitige, unerwünschte Reaktion der Inhaltsstoffe bzw. eine vorzeitige Zersetzung des Oxidationsmittels in basischem Milieu zu verhindern, werden Oxidationsmittel selbst und Alkalisierungsmittel zweckmäßigerweise getrennt voneinander konfektioniert und erst unmittelbar vor der Anwendung in Kontakt gebracht. Üblicherweise werden Aufhell- bzw. Bleichmittel daher in Form eines aus zwei Komponenten bestehenden "Kits" (Mehrkomponenten-Verpackungseinheit) angeboten, wobei die erste Komponente das Oxidationsmittel und die zweite Komponente das Alkalisierungsmittel (beispielsweise Ammoniak) enthält.

In einer weiteren Ausführungsform der vorliegenden Erfindung sind daher kosmetische Mittel (M1) bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden und wobei ein Container eine Oxidationsmittelzubereitung (M1a), enthaltend mindestens ein Oxidationsmittel, enthält und ein weiterer Container eine Alkalisierungsmittlezubereitung (M1b) enthält, welche in einem kosmetisch verträglichen Träger mindestens ein Alkalisierungsmittel umfasst..

Die Oxidationsmittelzubereitung (M1a) enthält bevorzugt die im Zusammenhang mit dem kosmetischen Mittel (M1) aufgeführten Oxidationsmittel in Form von Wasserstoffperoxid und/oder eines seiner festen Anlagerungsprodukte an organische oder anorganische Verbindungen, wie Harnstoff, Melamin sowie Natriumborat und gegebenenfalls mindestens einen zuvor im Zusammenhang mit dem kosmetischen Mittel (M1) aufgeführten Wirk-, Hilfs- oder Zusatzstoff.

Es hat sich als vorteilhaft erwiesen, wenn die Oxidationsmittelzubereitungen (M1a) zur Stabilisierung des Oxidationsmittels, insbesondere des Wasserstoffperoxids, zusätzlich mindestens einen zuvor genannten Stabilisator oder Komplexbildner enthalten. Besonders bevorzugte Stabilisatoren sind insbesondere EDTA und EDDS sowie Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Die Alkalisierungsmittelzubereitung (M1b) enthält bevorzugt die im Zusammenhang mit dem kosmetischen Mittel (M1) aufgeführten Alkalisierungsmittel in Form von Natriumhydroxid, Kaliumhydroxid, Ammoniak, Monoethanolamin und 2-Amino-2-methylpropanol und gegebenenfalls mindestens einen zuvor im Zusammenhang mit dem kosmetischen Mittel (M1) aufgeführten Wirk-, Hilfs- oder Zusatzstoff. Erfindungsgemäß besonders bevorzugt ist daher das Alkalisierungsmittel in der Alkalisierungsmittelzubereitung (M1b) ausgewählt aus der Gruppe von Natriumhydroxid, Kaliumhydroxid, Ammoniak, Monoethanolamin und 2-Amino-2-methylpropan sowie deren Mischungen und ist in einer Gesamtmenge von 0,2 bis 9,0 Gew.-%, vorzugsweise von 0,3 bis 8,0 Gew.-%, bevorzugt von 0,4 bis 7,0 Gew.-%, insbesondere von 0,5 bis 6,5 Gew.-%, bezogen auf das Gesamtgewicht der Alkalisierungsmittelzubereitung (M1b), enthalten.

In Verfahrensschritt c) des erfindungsgemäßen Verfahrens wird ein kosmetisches Mittel (M2) auf die keratinischen Fasern appliziert, welche noch mit dem Mittel (M1) beaufschlagt sind. Dieses kosmetische Mittel (M2), welches im nachfolgenden auch als Färbemittel bezeichnet wird, enthält mindestens ein Oxidationsfarbstoffvorprodukt vom Entwicklertyp (M2-1), mindestens ein Oxidationsfarbstoffvorprodukt vom Kupplertyp (M2-1) und mindestens ein Oxidationsmittel (M2-3).

Bevorzugte kosmetische Mittel (M2) enthalten mindestens ein Oxidationsfarbstoffvorprodukt vom Entwickleryp. Entsprechende erfindungsgemäße Verfahren, bei welchen das kosmetische Mittel (M2) als Oxidationsfarbstoffvorprodukt (M2-1) ein oder mehrere Oxidationsfarbstoffvorprodukte vom Entwicklertyp enthält, sind erfindungsgemäß bevorzugt. Erfindungsgemäß bevorzugt ist das mindestens eine Oxidationsfarbstoffvorprodukt vom Entwicklertyp (M2-1) ausgewählt aus der Gruppe von 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1 -on, p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol sowie deren physiologisch verträglichen Salzen und deren Mischungen.

Um natürliche Färbungen zu erhalten, müssen üblicherweise mehrere Oxidationsfarbstoffvorprodukte vom Entwicklertyp eingesetzt werden. Bevorzugte kosmetische Mittel (M2) sind daher dadurch gekennzeichnet, dass das mindestens eine Oxidationsfarbstoffvorprodukt vom Entwicklertyp (M2-1) ausgewählt ist aus mindestens einer der folgenden Kombinationen: p-Toluylendiamin/2-(2-Hydroxyethyl)-p-phenylendiamin; p-Toluylendiamin/2-Methoxymethyl-p-phenylendiamin; p-Toluylendiamin/N,N-Bis-(2-hydroxyethyl)-p-phenylendiamin; p-Toluylendiamin/2-Methoxymethyl-p-phenylendiamin; p-Toluylendiamin/N-(4-Amino-3-methylphenyl)-N-[3-(1H-imidazol-1-yl)propyl]amin; p-Toluylendiamin/Bis-(2-hydroxy-5-aminophenyl)methan; p-Toluylendiamin/4-Amino-3-methylphenol; p-Toluylendiamin/4,5-Diamino-1-(2-hydroxyethyl)pyrazol; p-Toluylendiamin/2,4,5,6-Tetraaminopyrimidin; 2-(2-Hydroxyethyl)-p-phenylendiamin/2-Methoxymethyl-p-phenylendiamin; 2-(2-Hydroxyethyl)-p-phenylendiamin /N,N-Bis-(2-hydroxyethyl)-p-phenylendiamin; 2-(2-Hydroxyethyl)-p-phenylendiamin /2-Methoxymethyl-p-phenylendiamin; 2-(2-Hydroxyethyl)-p-phenylendiamin /N-(4-Amino-3-methylphenyl)-N-[3-(1H-imidazol-1-yl)propyl]amin; 2-(2-Hydroxyethyl)-p-phenylendiamin/Bis-(2-hydroxy-5-aminophenyl)methan; 2-(2-Hydroxyethyl)-p-phenylendiamin/4-Amino-3-methylphenol; 2-(2-Hydroxyethyl)-p-phenylendiamin /4,5-Diamino-1-(2-hydroxyethyl)pyrazol; 2-(2-Hydroxyethyl)-p-phenylendiamin /2,4,5,6-Tetraaminopyrimidin; 2-Methoxymethyl-p-phenylendiamin/2-(2-Hydroxyethyl)-p-phenylendiamin; 2-Methoxymethyl-p-phenylendiamin /2-Methoxymethyl-p-phenylendiamin; 2-Methoxymethyl-p-phenylendiamin /N,N-Bis-(2-hydroxyethyl)-p-phenylendiamin; 2-Methoxymethyl-p-phenylendiamin /2-Methoxymethyl-p-phenylendiamin; 2-Methoxymethyl-p-phenylendiamin /N-(4-Amino-3-methylphenyl)-N-[3-(1H-imidazol-1-yl)-propyl]amin; /Bis-(2-hydroxy-5-aminophenyl)methan; 2-Methoxymethyl-p-phenylendiamin/4-Amino-3-methylphenol; 2-Methoxymethyl-p-phenylendiamin /4,5-Diamino-1-(2-hydroxyethyl)pyrazol; 2-Methoxymethyl-p-phenylendiamin /2,4,5,6-Tetraaminopyrimidin und/oder 4-und Amino-3-methylphenol/4,5-Diamino-1-(2-hydroxyethyl)pyrazol und/oder deren physiologisch verträgliche Salze.

Gemäß einer besonders bevorzugten Ausführungsform des ersten Erfindungsgegenstands ist das mindestens eine Oxidationsfarbstoffvorprodukt vom Entwicklertyp (M2-1) ausgewählt aus der Gruppe von p-Toluylendiamin, 4-Amino-3-methylphenol, 4,5-Diamino-1-(2-hydroxyethyl)pyrazol, 2,4,5,6-Tetraaminopyrimidin und/oder deren physiologisch verträglichen Salzen sowie deren Mischungen. Es hat sich gezeigt, dass der Einsatz dieser speziellen Oxidationsfarbstoffvorprodukte vom Entwicklertyp (M2-1) in den im Rahmen des erfindungsgemäßen Verfahrens eingesetzten Färbemitteln (M2) zu besonders lebendigen, wasch-, reib-, schweiß- und UV-echten multitonalen Färbungen führt.

Besonders ansprechend aussehende multitonale Färbungen werden erhalten, wenn das kosmetische Mittel (M2) das mindestens eine Oxidationsfarbstoffvorprodukt vom Entwicklertyp (M2-1) in einer Gesamtmenge von 0,0025 bis 10,0 Gew.-%, vorzugsweise von 0,004 bis 8,0 Gew.-%, bevorzugt 0,005 bis 5,0 Gew.-%, insbesondere 0,01 bis 3,5 Gew.-%, bezogen auf das Gesamtgewicht des kosmetischen Mittels (M2), enthält. Die vorstehend genannten Mengen der Entwicklerkomponente (M2-1) führen zu multitonalen Färbungen, welche besonders intensive und leuchtende Farben sowie eine hohe Beständigkeit gegen Umwelteinflüsse, wie Haarwäschen, UV-Licht, Schweiß und Reibung, aufweisen.

Das oxidative Färbemittel (M2) enthält als weiteren Bestandteil mindestens ein Oxidationsfarbstoffvorprodukt vom Kupplertyp (M2-2). Oxidationsfarbstoffvorprodukte vom Kupplertyp bilden im Rahmen der oxidativen Färbung allein keine signifikante Färbung aus, sondern benötigen für eine ausreichende Färbung die Gegenwart von Oxidationsfarbstoffvorprodukten vom Entwicklertyp. Oxidationsfarbstoffvorprodukte vom Kupplertyp im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Im Rahmen der vorliegenden Erfindung ist es bevorzugt, wenn das mindestens eine Oxidationsfarbstoffvorprodukt vom Kupplertyp (M2-2) ausgewählt ist aus der Gruppe von m-Aminophenol und dessen Derivaten, o-Aminophenol und dessen Derivaten, m-Diaminobenzol und dessen Derivaten, o-Diaminobenzol und dessen Derivaten, Di- und Trihydroxybenzolderivaten, Pyridinderivaten, Naphthalinderivaten, Morpholinderivaten, Chinoxalinderivaten, Pyrazolderivaten, Indolderivaten, Pyrimidinderivaten, Methylendioxybenzolderivaten und/oder deren physiologisch verträglichen Salzen sowie deren Mischungen.

Bevorzugte erfindungsgemäße Verfahren sind dadurch gekennzeichnet, dass das mindestens eine Oxidationsfarbstoffvorprodukt vom Kupplertyp (M2-2) ausgewählt ist aus der Gruppe von Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin, 1-Naphthol und/oder deren physiologisch verträglichen Salzen sowie deren Mischungen. Die vorstehend genannten Kupplerkomponenten (M2-2) führen in Kombination mit der mindestens einen Entwicklerkomponente (M2-1) in dem oxidativen Färbemittel (M2) zu besonders intensiven und beständigen multitonalen Färbeergebnissen.

Erfindungsgemäß bevorzugt enthält das kosmetische Mittel (M2) das mindestens eine Oxidationsfarbstoffvorprodukt vom Kupplertyp (M2-2) in einer Gesamtmenge von 0,0001 bis 6,0 Gew.-%, vorzugsweise von 0,001 bis 5,5 Gew.-%, bevorzugt von 0,002 bis 4,5 Gew.-%, insbesondere von 0,005 bis 2,5 Gew.-%, insbesondere von 0,01 bis 2,0 Gew.-%, bezogen auf das Gesamtgewicht des kosmetischen Mittels (M2). Die vorstehend genannten Mengen der Kupplerkomponente (M2-2) in den im Rahmen des erfindungsgemäßen Verfahrens eingesetzten oxidativen Färbemittels (M2) führen zu besonders lebendigen, wasch-, reib-, schweiß- und UV-echten multitonalen Färbungen.

Um eine ausgewogene und subtile Nuancenausbildung zu gewährleisten, können die im Rahmen des erfindungsgemäßen Verfahrens eingesetzten oxidativen Färbemittels (M2) zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Gemäß einer bevorzugten Ausführungsform des ersten Erfindungsgegenstands ist der mindestens eine direktziehende Farbstoff (M2) ausgewählt aus der Gruppe von anionischen direktziehenden Farbstoffen, kationischen direktziehenden Farbstoffen, nichtionischen direktziehenden Farbstoffen sowie deren Mischungen.

In diesem Zusammenhang kann es erfindungsgemäß vorgesehen sein, dass der anionische direktziehende Farbstoff ausgewählt ist aus der Gruppe von Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Tetrabromphenolblau und/oder deren physiologisch verträglichen Salzen.

Im Rahmen dieser Ausführungsform kann es weiterhin vorgesehen sein, dass der kationische direktziehende Farbstoff ausgewählt ist aus der Gruppe von Basic Blue 7, Basic Blue 26, Basic Violet 2 und Basic Violet 14, Basic Yellow 57, Basic Red 76, Basic Blue 99, Basic Brown 16 und Basic Brown 17 und HC Blue 16, sowie Basic Yellow 87, Basic Orange 31 und Basic Red 51 und/oder deren physiologisch verträglichen Salzen.

Darüber hinaus kann es im Rahmen dieser Ausführungsform auch vorgesehen sein, dass der nichtionische direktziehende Farbstoff ausgewählt ist aus der Gruppe von HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure, 2-Amino-6-chloro-4-nitrophenol, 4-Ethylamino-3-nitrobenzoesäure und 2-Chlor-6-ethylamino-4-nitrophenol und/oder deren physiologisch verträglichen Salzen, bevorzugt 2-Amino-6-chloro-4-nitrophenol und/oder 4-Amino-3-nitrophenol und/oder deren physiologisch verträglichen Salzen.

Im Rahmen des erfindungsgemäßen Verfahrens besonders bevorzugt eingesetzte oxidative Färbemittel (M2) enthalten zusätzlich mindestens feinen direktziehenden Farbstoff, welcher ausgewählt ist aus der Gruppe von 2-Amino-6-chloro-4-nitrophenol, HC Blue 12, HC Yellow 2, HC Violet 14D und/oder deren physiologisch verträglichen Salzen sowie deren Mischungen. Bei Einsatz dieser speziellen direktziehenden Farbstoffe wird eine besonders ausgewogene und subtile Nuancenbildung während des erfindungsgemäßen Verfahrens bzw. bei der multitonalen Färbung erreicht.

Erfindungsgemäß bevorzugt enthält das kosmetische Mittel (M2) zusätzlich mindestens einen direktziehenden Farbstoff in einer Gesamtmenge von 0,00001 bis 5,0 Gew.-%, vorzugsweise von 0,00005 bis 4,5 Gew.-%, bevorzugt von 0,0001 bis 4,0 Gew.-%, weiter bevorzugt von 0,0005 bis 3,5 Gew.-%, insbesondere von 0,001 bis 3,0 Gew.-%, bezogen auf das Gesamtgewicht des kosmetischen Mittels (M2). Die zuvor genannten Mengen der direktziehenden Farbstoffe führen zu besonders ausgewogenen Nuancen im Rahmen der multitonalen Färbung gemäß dem erfindungsgemäßen Verfahren.

Auch das kosmetische Mittel (M2) kann mindestens ein Alkalisierungsmittel enthalten. Geeignete Alkalisierungsmittel sowie deren einsetzbare Gesamtmengen sind bereits im Zusammenhang mit dem Vorbehandlungsmittel (M1) angeführt worden. Die Einstellung eines basischen pH-Werts unter Verwendung des mindestens einen Alkalisierungsmittels ist erforderlich, um eine Öffnung der äußeren Schuppenschicht (Cuticula) zu gewährleisten und eine Penetration der Oxidationsfarbstoffvorprodukte in das Haar zu ermöglichen.

Erfindungsgemäß bevorzugte Verfahren sind daher dadurch gekennzeichnet, dass das kosmetische Mittel (M2) einen pH-Wert von pH 5,0 bis pH 12, vorzugsweise von pH 5,0 bis pH 11, bevorzugt von pH 5,5 bis pH 10, insbesondere von pH 6,0 bis pH 9,0, aufweist.

Besonders lebendige multitonale Färbungen werden erzielt, ist es von Vorteil, wenn die sequentiell aufgetragenen kosmetischen Mittel (M1) und (M2) unterschiedliche pH-Werte aufweisen. Es sind daher erfindungsgemäße Verfahren bevorzugt, bei welchen das kosmetischen Mittel (M1) und das kosmetischen Mittel (M2) unterschiedliche pH-Werte aufweisen.

Die Färbemittel (M2) können weiterhin zusätzliche Wirk-, Hilfs- und Zusatzstoffe enthalten, um die Färbeleistung zu verbessern und weitere gewünschte Eigenschaften der kosmetischen Mittel (M2) einzustellen.

Erfindungsgemäß bevorzugt enthält das kosmetische Mittel (M2) daher zusätzlich mindestens eine weitere Verbindung, ausgewählt aus der Gruppe von (i) Verdickungsmitteln; (ii) Tensiden; (iii) kationischen Polymeren; (iv) Siliconen; (v) Alkalisierungsmitteln; sowie (vi) deren Mischungen.

Es sich als vorteilhaft erwiesen, wenn die kosmetischen Mittel (M2) ebenfalls mindestens ein Verdickungsmittel enthalten. Bezüglich dieser Verdickungsmittel bestehen keine prinzipiellen Einschränkungen. Geeignete Verdickungsmittel sind die im Zusammenhang mit dem Vorbehandlungsmittel (M1) angeführten Verbindungen, welche zur Verdickung der Färbemittel (M2) ebenfalls eingesetzt werden können. Daneben können auch die nachfolgend angeführten organischen und anorganischen Verdickungsmittel verwendet werden.

Geeignete Verdickungsmittel sind anionische, synthetische Polymere; kationische, synthetische Polymere sowie nichtionische, synthetische Polymere, wie Polyvinylalkohol oder Polyvinylpyrrolidon.

Die in dem erfindungsgemäßen Verfahren eingesetzten kosmetischen Mittel (M2) können als Verdickungsmittel auch zwitterionische Polymere enthalten, welche ausgewählt sind aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminiumsalzen und Acrylsäure,
- Copolymere aus N,N,N-Trimethyl-3-[(2-methyl-1-oxo-2-propen-1-yl)amino]-1-propanaminiumsalzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminiumsalzen, Acrylsäure und Acrylamid, z.B. Polyquaternium-53
- Copolymere aus N,N,N-Trimethyl-3-[(2-methyl-1-oxo-2-propen-1-yl)amino]-1-propanaminiumsalzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.

Auch Gemische der vorgenannten zwitterionischen Polymere können zur Verdickung der kosmetischen Mittel (M2) eingesetzt werden.

Vorzugsweise werden die Färbemittel (M2) als flüssige Zubereitung bereitgestellt und diesen Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Im Rahmen dieser Ausführungsform kann es vorgesehen sein, dass das anionische Tensid ausgewählt ist aus der Gruppe von Alkylsulfaten und Alkylpolyglykolethersulfaten der Formel R²-O(CH₂-CH₂O)ₓ-OSO₃H, in welcher R² eine lineare Alkylgruppe mit 8 bis 30 Kohlenstoffatomen und x = 0 oder 1 bis 12 ist, Salzen linearer und verzweigter Carbonsäuren mit 8 bis 30 Kohlenstoffatomen, Ethercarbonsäuren der Formel R³-O-(CH₂-CH₂O)ₓ-CH₂-COOH, in welcher R³ eine lineare Alkylgruppe mit 8 bis 30 C-Atomen und x = 0 oder 1 bis 16 ist, sowie deren Mischungen. Die anionischen Tenside werden bevorzugt in einer Gesamtmenge von 0,1 bis 45 Gew.-%, vorzugsweise von 1 bis 30 Gew.-%, insbesondere von 1 bis 15 Gew.-%, bezogen auf die Gesamtmenge des kosmetischen Mittels (M2), eingesetzt.

In diesem Zusammenhang kann es erfindungsgemäß weiterhin vorgesehen sein, dass das nichtionische Tensid ausgewählt ist aus der Gruppe von ethoxylierten Alkoholen und Carbonsäuren mit 8 bis 13 Kohlenstoffatomen und 2 bis 30 Ethylenoxideinheiten, Anlagerungsprodukten von 5 bis 60 Mol Ethylenoxid an Rizinusöl und gehärtetes Rizinusöl, Alkylpolyglucosiden der Formel R¹O-[G]ₚ, in welcher R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, Monoethanolamiden von Carbonsäuren 8 bis 30 Kohlenstoffatomen sowie deren Mischungen.

Darüber hinaus kann es im Rahmen dieser Ausführungsform auch vorgesehen sein, dass das amphotere Tensid ausgewählt ist aus der Gruppe von Amphoacetaten mit Carbonsäureresten mit 8 bis 30 Kohlenstoffatomen, N-Alkylglycinen, N-Alkylpropionsäuren, N-Alkylamidobuttersäuren, N-Alkyliminodipropionsäuren, N-Hydroxyethyl-N-alkylamidopropylglycinen, N-Alkyltaurinen, N-Alkylsarcosinen, 2-Alkylaminopropionsäuren, Alkylaminoessigsäuren sowie deren Mischungen.

Im Rahmen dieser Ausführungsform kann es zudem vorgesehen sein, dass das zwitterionische Tensid ausgewählt ist aus der Gruppe von Betainen, N-Alkyl-N,N-diemthylammoniumglycinaten, N-Acyl-amidopropyl-N,N-dimethylammoniumglycinaten, 2-Alkyl-3-carboxymethyl-3-hydroxyethyl-imidazolinen sowie deren Mischungen.

Die nichtionischen und/oder zwitterionischen und/oder amphoteren Tenside werden bevorzugt in einer Gesamtmenge von 0,1 bis 45 Gew.-%, vorzugsweise von 1 bis 30 Gew.-%, insbesondere von 1 bis 15 Gew.-%, bezogen auf die Gesamtmenge des kosmetischen Mittels (M2), eingesetzt.

Weiterhin kann der Einsatz von kationischen Tensiden, kationischen Polymeren sowie Silikonen in dem kosmetischen Mittel (M2) vorgesehen sein. Geeignete kationische Tenside, kationische Polymere und Silikone sowie die in den kosmetischen Mitteln (M2) enthaltenen Gesamtmengen sind bereits im Zusammenhang mit dem Vorbehandlungsmittel (M1) aufgeführt.

Auch das kosmetische Mittel (M2) kann mindestens ein Alkalisierungsmittel enthalten. Geeignete Alkalisierungsmittel sowie deren einsetzbare Gesamtmengen sind bereits im Zusammenhang mit dem Vorbehandlungsmittel (M1) angeführt worden. Besonders bevorzugt ist das Alkalisierungsmittel ausgewählt aus der Gruppe von Natriumhydroxid, Kaliumhydroxid, Ammoniak, Monoethanolamin und 2-Amino-2-methylpropan sowie deren Mischungen und ist in einer Gesamtmenge von 0,05 bis 8,0 Gew.-%, vorzugsweise von 0,1 bis 6,0 Gew.-%, bevorzugt von 0,3 bis 5,5 Gew.-%, insbesondere von 0,5 bis 5,0 Gew.-%, bezogen auf das Gesamtgewicht des kosmetischen Mittels (M2), enthalten. Die Einstellung eines basischen pH-Werts unter Verwendung des mindestens einen Alkalisierungsmittels ist erforderlich, um eine Öffnung der äußeren Schuppenschicht (Cuticula) zu gewährleisten und eine Penetration der Oxidationsfarbstoffvorprodukte in das Haar zu ermöglichen.

Die Oxidationsfarbstoffvorprodukte (Entwickler und Kuppler) selbst sind nicht gefärbt. Die Bildung der eigentlichen Farbstoffe erfolgt erst im Verlauf der Anwendung durch den Kontakt der Oxidationsfarbstoffvorprodukte mit einem Oxidationsmittel (vorzugsweise Wasserstoffperoxid). In einer chemischer Reaktion werden die als Oxidationsfarbstoffvorprodukte eingesetzten Entwickler (wie beispielsweise p-Phenylendiamin-Derivate oder p-Aminophenolderivate) durch Wasserstoffperoxid zunächst oxidativ in eine reaktive Zwischenstufe, auch Chinonimin oder Chinondiimin genannt, überführt, welche dann in einer oxidativen Kupplungsreaktion mit den Kupplern zum jeweiligen Farbstoff reagiert.

Die kosmetischen Mittel (M2) enthalten daher zusätzlich ein oder mehrere Oxidationsmittel (M2-3). Als Oxidationsmittel kommen Persulfate, Peroxodisulfate, Chlorite, Hypochlorite und insbesondere Wasserstoffperoxid und/oder eines seiner festen Anlagerungsprodukte an organische oder anorganische Verbindungen in Frage.

Erfindungsgemäß bevorzugte Verfahren sind daher dadurch gekennzeichnet, dass das kosmetische Mittel (M2) mindestens ein Oxidationsmittel (M2-3) aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat, bevorzugt Wasserstoffperoxid, in einer Gesamtmenge von 0,1 bis 6 Gew.-%, vorzugsweise von 0,3 bis 4 Gew.-%, insbesondere von 0,5 bis 3 Gew.-%, bezogen auf das Gesamtgewicht des kosmetischen Mittels (M2), enthält. Falls als Oxidationsmittel Wasserstoffperoxid sowie dessen feste Anlagerungsprodukte eingesetzt werden, berechnet sich die vorstehend genannte Gesamtmenge auf 100%iges H₂O₂.

In einer weiteren bevorzugten Ausführungsform ist das kosmetische Mittel (M2) ein Mittel zum Färben und gegebenenfalls gleichzeitigen Aufhellen keratinischer Fasern, welches Wasserstoffperoxid in einer Gesamtmenge von 0,5 bis 15 Gew.-%, vorzugsweise 1,0 bis 12,5 Gew.-%, bevorzugt 1,5 bis 10 Gew.-%, insbesondere 2,0 bis 6,5 Gew.-%, bezogen auf das Gesamtgewicht des kosmetischen Mittels (M2), enthält. Die vorstehend genannte Gesamtmenge an Wasserstoffperoxid ist hierbei auf 100%iges H₂O₂ bezogen.

Zur Erzielung einer verstärkten Aufhell- und Bleichwirkung kann das kosmetische Mittel (M2) weiterhin mindestens ein Peroxo-Salz enthalten. Geeignete Peroxo-Salze sind anorganische Peroxoverbindungen, bevorzugt ausgewählt aus der Gruppe von Ammoniumperoxodisulfat, Alkalimetallperoxodisulfaten, Ammoniumperoxomonosulfat, Alkalimetallperoxomonosulfaten, Alkalimetallperoxodiphosphaten, Erdalkalimetallperoxiden sowie deren Mischungen. Besonders bevorzugt sind Peroxodisulfate, insbesondere Ammoniumperoxodisulfat, Kaliumperoxodisulfat und Natriumperoxodisulfat.

Die zuvor genannten Peroxosalze sind in einer Gesamtmenge von 0,5 bis 20 Gew.-%, vorzugsweise 1 bis 12,5 Gew.-%, bevorzugt 2,5 bis 10 Gew.-%, insbesondere 3 bis 6 Gew.-%, bezogen auf das Gesamtgewicht des kosmetischen Mittels (M2), enthalten.

Vorzugsweise enthält das Aufhell- bzw. Bleichmittel (M1) das Oxidationsmittel (M1-1) in einer höheren Gesamtstoffmenge als das kosmetische Mittel (M2). Dies führt zu besonders intensiven und lebendigen multitonalen Färbungen, welche darüber hinaus eine hohe Beständigkeit gegen Umwelteinflüsse, wie beispielsweise Haarwäschen, Schweiß, UV-Licht oder Reibung, aufweisen.

Gemäß einer besonders bevorzugten Ausführungsform der vorliegenden Erfindung weist daher das Mengenverhältnis der Gesamtmenge des Oxidationsmittels (M1-1) in dem kosmetischen Mittel (M1) zur Gesamtmenge des Oxidationsmittels (M2-3) in dem kosmetischen Mittel (M2) einen Wert (M1-1)/(M2-3) von 16:1 bis 1:1, vorzugsweise von 8:1 bis 1:1, bevorzugt von 6:1 bis 1:1, weiter bevorzugt noch 5 :1 bis 1:1, insbesondere von 4:1 bis 1:1, auf.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Üblicherweise werden oxidative Färbemittel daher in Form eines aus zwei Komponenten bestehenden "Kits" (Mehrkomponenten-Verpackungseinheit) angeboten, wobei die erste Komponente die Oxidationsfarbstoffvorprodukte und gegebenenfalls direktziehenden Farbstoffen sowie ein Alkalisierungsmittel (beispielsweise Ammoniak) und die zweite Komponente das Oxidationsmittel enthält.

In einer weiteren Ausführungsform der vorliegenden Erfindung sind daher kosmetische Mittel (M2) bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden und wobei ein Container ein Färbemittel (M2a), welches in einem kosmetischen Träger mindestens ein Oxidationsfarbstoffvorprodukt enthält, und ein weiterer Container eine Oxidationsmittelzubereitung (M2b), enthaltend mindestens ein Oxidationsmittel, enthält.

Das Färbemittel (M2a) enthält hierbei bevorzugt die zuvor im Zusammenhang mit dem kosmetischen Mittel (M2) aufgeführten Oxidationsfarbstoffvorprodukte vom Entwicklertyp und/oder Kupplertyp, gegebenenfalls mindestens einen direktziehenden Farbstoff und gegebenenfalls mindestens einen zuvor im Zusammenhang mit dem kosmetischen Mittel (M2) aufgeführten Wirk-, Hilfs- oder Zusatzstoff. Die Oxidationsmittelzubereitung (M2b) enthält bevorzugt ein Oxidationsmittel in Form von Wasserstoffperoxid und/oder eines seiner festen Anlagerungsprodukte an organische oder anorganische Verbindungen, wie Harnstoff, Melamin sowie Natriumborat.

Solche Oxidationsmittelzubereitungen (M2b) sind vorzugsweise wässrige, fließfähige Oxidationsmittelzubereitungen. Dabei sind bevorzugte Zubereitungen (M2b) dadurch gekennzeichnet, dass die fließfähige Oxidationsmittelzubereitung (M2b) 40 bis 90 Gew.-%, vorzugsweise 50 bis 90 Gew.-%, bevorzugt 55 bis 89 Gew.-%, weiter bevorzugt 60 bis 87 Gew.-%, insbesondere 65 bis 85 Gew.-%, bezogen auf das Gesamtgewicht der Oxidationsmittelzubereitung (M2b), Wasser enthält.

Bevorzugt beträgt die Gesamtmenge an Oxidationsmittel, insbesondere Wasserstoffperoxid, in der Oxidationsmittelzubereitung (M2b) 0,5 bis 12 Gew.-%, vorzugsweise 1,0 bis 10 Gew.-%, insbesondere 1,5 bis 6,0 Gew.-%, bezogen auf das Gesamtgewicht der Oxidationsmittelzubereitung (M2b). Falls als Oxidationsmittel Wasserstoffperoxid sowie dessen feste Anlagerungsprodukte eingesetzt werden, berechnet sich die vorstehend genannte Gesamtmenge auf 100%iges H₂O₂.

Erfindungsgemäß kann die Oxidationsmittelzubereitung (M2b) auch zusammen mit einem Katalysator auf das Haar aufgebracht werden, welcher die Oxidation der Farbstoffvorprodukte aktiviert. Solche Katalysatoren sind z. B. bestimmte Enzyme, lodide, Chinone oder Metallionen.

Es hat sich als vorteilhaft erwiesen, wenn die Oxidationsmittelzubereitungen (M2b) zur Stabilisierung des Oxidationsmittels, insbesondere des Wasserstoffperoxids, zusätzlich mindestens einen Stabilisator oder Komplexbildner enthalten. Besonders bevorzugte Stabilisatoren sind insbesondere EDTA und EDDS sowie Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

## Patentansprüche

1. Verfahren zum oxidativen Färben von keratinischen Fasern, wobei das Verfahren die folgenden Verfahrensschritte umfasst:
a) Applikation eines kosmetischen Mittels (M1) auf die keratinischen Fasern, wobei das kosmetische Mittel (M1) nur auf einzelne Strähnchen appliziert wird,
b) Einwirkenlassen des Mittels (M1) für einen Zeitraum von 5 bis 30 Minuten auf den keratinischen Fasern,
c) Applikation eines kosmetischen Mittels (M2) auf die mit dem kosmetischen Mittel (M1) beaufschlagten keratinischen Fasern,
d) Einwirkenlassen der kosmetischen Mittel (M1) und (M2) für einen Zeitraum von 1 bis 70 Minuten auf den keratinischen Fasern,
e) Ausspülen der kosmetischen Mittel (M1) und (M2),
**dadurch gekennzeichnet, dass**
- das kosmetische Mittel (M1)
mindestens ein Oxidationsmittel enthält (M1-1),
einen pH-Wert von pH 7 bis pH 14 aufweist (M1-2),
keine Verbindung aus der Gruppe der Oxidationsfarbstoffvorprodukte vom Entwicklertyp und Kupplertyp, der direktziehenden Farbstoffe sowie deren Mischungen enthält (M1-3), und
eine dynamische Viskosität von 10.000 bis 50.000 mPa*s, jeweils gemessen mit Brookfield RDV II+, Spindel Nr. 4, 4 rpm, 20 °C, aufweist,
- das kosmetische Mittel (M2)
mindestens ein Oxidationsfarbstoffvorprodukt vom Entwicklertyp enthält (M2-1),
mindestens ein Oxidationsfarbstoffvorprodukt vom Kupplertyp enthält (M2-2),
mindestens ein Oxidationsmittel enthält (M2-3), und
einen pH-Wert von pH 5 bis pH 12 aufweist (M2-4).

2. Verfahren nach Anspruch 1, **dadurch gekennzeichnet, dass** die kosmetischen Mittel (M1) und (M2) in Verfahrensschritt d) für einen Zeitraum von 1 bis 60 Minuten, vorzugsweise von 5 bis 50 Minuten, insbesondere von 10 bis 45 Minuten, Einwirken gelassen werden.

3. Verfahren nach einem der vorangehenden Ansprüche, **dadurch gekennzeichnet, dass** das kosmetische Mittel (M1) das mindestens eine Oxidationsmittel (M1-1) in einer Gesamtmenge von 1,0 bis 8,0 Gew.-%, vorzugsweise von 1,5 bis 7,5 Gew.-%, bevorzugt von 2,0 bis 7,0 Gew.-%, weiter bevorzugt von 2,5 bis 7,0 Gew.-%, insbesondere von 3,0 bis 7,0 Gew.-%, bezogen auf das Gesamtgewicht des kosmetischen Mittels (M1), enthält.

4. Verfahren nach einem der vorangehenden Ansprüche, **dadurch gekennzeichnet, dass** das kosmetische Mittel (M1) als Oxidationsmittel (M1-1) Wasserstoffperoxid oder dessen feste Anlagerungsprodukte an Harnstoff, Melamin, Polyvinylpyrrolidon und Natriumborat, bevorzugt Wasserstoffperoxid in einer Gesamtmenge von 1,0 bis 12 Gew.-%, vorzugsweise von 1,5 bis 12 Gew.-%, bevorzugt von 2,0 bis 12 Gew.-%, weiter bevorzugt von 3,0 bis 12 Gew.-%, insbesondere von 3,5 bis 12,0 Gew.-%, bezogen auf das Gesamtgewicht des kosmetischen Mittels (M1), enthält.

5. Verfahren nach einem der vorangehenden Ansprüche, **dadurch gekennzeichnet, dass** das kosmetische Mittel (M1) einen pH-Wert (M1-2) von pH 7,5 bis pH 13, vorzugsweise von pH 8,0 bis pH 12,5, bevorzugt von pH 8,0 bis pH 12, weiter bevorzugt von pH 8,0 bis pH 11,5, insbesondere von pH 8,0 bis pH 11, aufweist.

6. Verfahren nach einem der vorangehenden Ansprüche, **dadurch gekennzeichnet, dass** das mindestens eine Oxidationsfarbstoffvorprodukt vom Entwicklertyp (M2-1) ausgewählt ist aus der Gruppe von p-Toluylendiamin, 4-Amino-3-methylphenol, 4,5-Diamino-1-(2-hydroxyethyl)pyrazol, 2,4,5,6-Tetraaminopyrimidin und/oder deren physiologisch verträglichen Salzen sowie deren Mischungen.

7. Verfahren nach einem der vorangehenden Ansprüche, **dadurch gekennzeichnet, dass** das kosmetische Mittel (M2) das mindestens eine Oxidationsfarbstoffvorprodukt vom Entwicklertyp (M2-1) in einer Gesamtmenge von 0,0025 bis 10,0 Gew.-%, vorzugsweise von 0,004 bis 8,0 Gew.-%, bevorzugt 0,005 bis 5,0 Gew.-%, insbesondere 0,01 bis 3,5 Gew.-%, bezogen auf das Gesamtgewicht des kosmetischen Mittels (M2), enthält.

8. Verfahren nach einem der vorangehenden Ansprüche, **dadurch gekennzeichnet, dass** das mindestens eine Oxidationsfarbstoffvorprodukt vom Kupplertyp (M2-2) ausgewählt ist aus der Gruppe von Resorcin, 2-Methylresorcin, 4-Chlorresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin, 1-Naphthol, 2-Amino-4-hydroxyethylaminoanisol und/oder deren physiologisch verträglichen Salzen sowie deren Mischungen.

9. Verfahren nach einem der vorangehenden Ansprüche, **dadurch gekennzeichnet, dass** das kosmetische Mittel (M2) das mindestens eine Oxidationsfarbstoffvorprodukt vom Kupplertyp (M2-2) in einer Gesamtmenge von 0,0001 bis 6,0 Gew.-%, vorzugsweise von 0,001 bis 5,5 Gew.-%, bevorzugt von 0,002 bis 4,5 Gew.-%, insbesondere von 0,005 bis 2,5 Gew.-%, bezogen auf das Gesamtgewicht des kosmetischen Mittels (M2), enthält.

10. Verfahren nach einem der vorangehenden Ansprüche, **dadurch gekennzeichnet, dass** das kosmetische Mittel (M2) einen pH-Wert von pH 5,0 bis pH 12, vorzugsweise von pH 5,0 bis pH 11, bevorzugt von pH 5,5 bis pH 10, insbesondere von pH 6,0 bis pH 9,0, aufweist.

11. Verfahren nach einem der vorangehenden Ansprüche, **dadurch gekennzeichnet, dass** das kosmetische Mittel (M2) mindestens ein Oxidationsmittel (M2-3) aus der Gruppe von Persulfaten, Peroxodisulfaten, Chloriten, Hypochloriten, Wasserstoffperoxid sowie dessen feste Anlagerungsprodukte an Harnstoff, Melamin, Polyvinylpyrrolidon und Natriumborat, bevorzugt Wasserstoffperoxid, in einer Gesamtmenge von 0,1 bis 6 Gew.-%, vorzugsweise von 0,3 bis 4 Gew.-%, insbesondere von 0,5 bis 3 Gew.-%, bezogen auf das Gesamtgewicht des kosmetischen Mittels (M2), enthält.

12. Verfahren nach einem der vorangehenden Ansprüche, **dadurch gekennzeichnet, dass** das Mengenverhältnis der Gesamtmenge des Oxidationsmittels (M1-1) in dem kosmetischen Mittel (M1) zur Gesamtmenge des Oxidationsmittels (M2-3) in dem kosmetischen Mittel (M2) einen Wert (M1-1)/(M2-3) von 16:1 bis 1:1, vorzugsweise von 8:1 bis 1:1, bevorzugt von 6:1 bis 1:1, weiter bevorzugt noch 5:1 bis 1:1, insbesondere von 4:1 bis 1:1, aufweist.

## Claims

1. A method for oxidatively dyeing keratin fibers, the method comprising the following method steps:
a) applying a cosmetic agent (M1) to the keratin fibers, the cosmetic agent (M1) being applied only to individual strands,
b) allowing the agent (M1) to act on the keratin fibers for a period of 5 to 30 minutes,
c) applying a cosmetic agent (M2) to the keratin fibers subjected to cosmetic agent (M1),
d) allowing the cosmetic agents (M1) and (M2) to act on the keratin fibers for a period of 1 to 70 minutes,
e) rinsing out the cosmetic agents (M1) and (M2),
**characterized in that**
- cosmetic agent (M1)
contains at least one oxidizing agent (M1-1),
has a pH of pH 7 to pH 14 (M1-2),
does not contain any compounds from the group of developer-type and coupler-type oxidation dye precursors, substantive dyes and mixtures thereof (M1-3), and has a dynamic viscosity of 10,000 to 50,000 mPa*s, measured using Brookfield RDV II+, spindle no. 4, 4 rpm, 20 °C,
- cosmetic agent (M2)
contains at least one developer-type oxidation dye precursor (M2-1),
contains at least one coupler-type oxidation dye precursor (M2-2),
contains at least one oxidizing agent (M2-3), and
has a pH of pH 5 to pH 12 (M2-4).

2. The method according to claim 1, **characterized in that** in method step d) the cosmetic agents (M1) and (M2) are allowed to act for a period of 1 to 60 minutes, preferably of 5 to 50 minutes, in particular of 10 to 45 minutes.

3. The method according to one of the preceding claims, **characterized in that** cosmetic agent (M1) contains the at least one oxidizing agent (M1-1) in a total amount of 1.0 to 8.0 wt.%, preferably 1.5 to 7.5 wt.%, more preferably 2.0 to 7.0 wt.%, even more preferably 2.5 to 7.0 wt.%, in particular 3.0 to 7.0 wt.%, based on the total weight of cosmetic agent (M1).

4. The method according to one of the preceding claims, **characterized in that** cosmetic agent (M1) contains, as the oxidizing agent (M1-1), hydrogen peroxide or the solid addition products thereof with urea, melamine, polyvinylpyrrolidone and sodium borate, preferably hydrogen peroxide in a total amount of 1.0 to 12 wt.%, preferably 1.5 to 12 wt.%, more preferably 2.0 to 12 wt.%, even more preferably 3.0 to 12 wt.%, in particular 3.5 to 12.0 wt.%, based on the total weight of cosmetic agent (M1).

5. The method according to one of the preceding claims, **characterized in that** cosmetic agent (M1) has a pH (M1-2) of pH 7.5 to pH 13, preferably of pH 8.0 to pH 12.5, more preferably of pH 8.0 to pH 12, even more preferably of pH 8.0 to pH 11.5, in particular of pH 8.0 to pH 11.

6. The method according to one of the preceding claims, **characterized in that** the at least one developer-type oxidation dye precursor (M2-1) is selected from the group of p-toluenediamine, 4-amino-3-methylphenol, 4,5-diamino-1-(2-hydroxyethyl)pyrazole, 2,4,5,6-tetraaminopyrimidine, and/or the physiologically acceptable salts thereof, and mixtures thereof.

7. The method according to one of the preceding claims, **characterized in that** cosmetic agent (M2) contains the at least one developer-type oxidation dye precursor (M2-1) in a total amount of 0.0025 to 10.0 wt.%, preferably of 0.004 to 8.0 wt.%, more preferably 0.005 to 5.0 wt.%, in particular 0.01 to 3.5 wt.%, based on the total weight of cosmetic agent (M2).

8. The method according to one of the preceding claims, **characterized in that** the at least one coupler-type oxidation dye precursor (M2-2) is selected from the group of resorcinol, 2-methylresorcinol, 4-chlororesorcinol, 5-amino-2-methylphenol, 3-aminophenol, 2-(2,4-diaminophenoxy)ethanol, 1,3-bis-(2,4-diaminophenoxy)propane, 1-methoxy-2-amino-4-(2'-hydroxyethylamino)benzene, 2-amino-3-hydroxypyridine, 2,6-dihydroxy-3,4-dimethylpyridine, 1,5-dihydroxynaphthalene, 2,7-dihydroxynaphthalene, 1-naphthol, 2-amino-4-hydroxyethylaminoanisole, and/or the physiologically acceptable salts thereof, and mixtures thereof.

9. The method according to one of the preceding claims, **characterized in that** cosmetic agent (M2) contains the at least one coupler-type oxidation dye precursor (M2-2) in a total amount of 0.0001 to 6.0 wt.%, preferably of 0.001 to 5.5 wt.%, more preferably of 0.002 to 4.5 wt.%, in particular of 0.005 to 2.5 wt.%, based on the total weight of cosmetic agent (M2).

10. The method according to one of the preceding claims, **characterized in that** cosmetic agent (M2) has a pH of pH 5.0 to pH 12, preferably of pH 5.0 to pH 11, more preferably of pH 5.5 to pH 10, in particular of pH 6.0 to pH 9.0.

11. The method according to one of the preceding claims, **characterized in that** cosmetic agent (M2) contains at least one oxidizing agent (M2-3) from the group of persulfates, peroxydisulfates, chlorites, hypochlorites, hydrogen peroxide and the solid addition products thereof with urea, melamine, polyvinylpyrrolidone and sodium borate, preferably hydrogen peroxide, in a total amount of 0.1 to 6 wt.%, preferably of 0.3 to 4 wt.%, in particular of 0.5 to 3 wt.%, based on the total weight of cosmetic agent (M2).

12. The method according to one of the preceding claims, **characterized in that** the quantitative ratio of the total amount of the oxidizing agent (M1-1) in cosmetic agent (M1) to the total amount of the oxidizing agent (M2-3) in cosmetic agent (M2) has a value (M1-1)/(M2-3) of 16:1 to 1:1, preferably of 8:1 to 1:1, more preferably of 6:1 to 1:1, even more preferably 5:1 to 1:1, in particular 4:1 to 1:1.

## Revendications

1. Procédé de coloration par oxydation de fibres kératiniques, le procédé comprenant les étapes de procédé suivantes :
a) application d'un agent cosmétique (M1) sur les fibres kératiniques, l'agent cosmétique (M1) n'étant appliqué que sur des mèches individuelles,
b) réaction de l'agent (M1) sur les fibres kératiniques pendant une durée de 5 à 30 minutes,
c) application d'un agent cosmétique (M2) sur les fibres kératiniques exposées à l'agent cosmétique (M1),
d) réaction des agents cosmétiques (M1) et (M2) sur les fibres kératiniques pendant une durée de 1 à 70 minutes,
e) rinçage des agents cosmétiques (M1) et (M2), **caractérisé en ce que**
- l'agent cosmétique (M1)
contient au moins un agent oxydant (M1-1),
présente un pH compris entre 7 et 14 (M1-2),
ne comporte aucun composé du groupe des précurseurs de colorant par oxydation du type développeur ou du type coupleur contenant des colorants directs ainsi que leurs mélanges (M1-3), et une viscosité dynamique de 10 000 à 50 000 mPa*s, mesurée dans chaque cas avec un viscosimètre Brookfield RDV II+, broche n°4, 4 tours par minute, 20 °C,
- l'agent cosmétique (M2)
contient au moins un précurseur de colorant par oxydation du type développeur (M2-1),
contient au moins un précurseur de colorant par oxydation du type coupleur (M2-2),
contient au moins un agent oxydant (M2-3), et
présente un pH compris entre 5 et 12 (M2-4).

2. Procédé selon la revendication 1, **caractérisé en ce qu'**on laisse agir les agents cosmétiques (M1) et (M2) à l'étape de procédé d) pendant une durée de 1 à 60 minutes, de préférence de 5 à 50 minutes, en particulier de 10 à 45 minutes.

3. Procédé selon l'une des revendications précédentes, **caractérisé en ce que** l'agent cosmétique (M1) contient l'au moins un agent oxydant (M1-1) en une quantité totale allant de 1,0 à 8,0 % en poids, de préférence de 1,5 à 7,5 % en poids, préférablement de 2,0 à 7,0 % en poids, de manière davantage préférée de 2,5 à 7,0 % en poids, en particulier de 3,0 à 7,0 % en poids, par rapport au poids total de l'agent cosmétique (M1).

4. Procédé selon l'une des revendications précédentes, **caractérisé en ce que** l'agent cosmétique (M1) contient, en tant qu'agent oxydant (M1-1), du peroxyde d'hydrogène ou ses produits de dépôt solides sur l'urée, la mélamine, la polyvinylpyrrolidone et le borate de sodium, de préférence du peroxyde d'hydrogène, en une quantité totale allant de 1,0 à 12 % en poids, de préférence de 1,5 à 12 % en poids, préférablement de 2,0 à 12 % en poids, de manière davantage préférée de 3,0 à 12 % en poids, en particulier de 3,5 à 12,0 % en poids, par rapport au poids total de l'agent cosmétique (M1).

5. Procédé selon l'une des revendications précédentes, **caractérisé en ce que** l'agent cosmétique (M1) présente un pH (M1-2) de 7,5 à 13, de préférence de 8,0 à 12,5, préférablement de 8,0 à 12, de manière davantage préférée de 8,0 à 11,5, en particulier de 8,0 à 11.

6. Procédé selon l'une des revendications précédentes, **caractérisé en ce que** l'au moins un précurseur de colorant par oxydation du type développeur (M2-1) est choisi dans le groupe constitué de la p-toluènediamine, du 4-amino-3-méthylphénol, du 4,5-diamino-1-(2-hydroxyéthyl)pyrazol, de la 2,4,5,6-tétraaminopyrimidine et/ou de leurs sels physiologiquement acceptables ainsi que leurs mélanges.

7. Procédé selon l'une des revendications précédentes, **caractérisé en ce que** l'agent cosmétique (M2) contient l'au moins un précurseur de colorant par oxydation du type développeur (M2-1) en une quantité totale allant de 0,0025 à 10,0 % en poids, de préférence de 0,004 à 8,0 % en poids, préférablement de 0,005 à 5,0 % en poids, en particulier de 0,01 à 3,5 % en poids, par rapport au poids total de l'agent cosmétique (M2).

8. Procédé selon l'une des revendications précédentes, **caractérisé en ce que** l'au moins un précurseur de colorant par oxydation du type coupleur (M2-2) est choisi dans le groupe constitué du résorcinol, du 2-méthylrésorcinol, du 4-chlororésorcinol, du 5-amino-2-méthylphénol, du 3-aminophénol, du 2-(2,4-diaminophénoxy)éthanol, du 1,3-bis-(2,4-diaminophénoxy)propane, du 1-méthoxy-2-amino-4-(2'-hydroxyéthylamino)benzène, de la 2-amino-3-hydroxypyridine, de la 2,6-dihydroxy-3,4-diméthylpyridine, du 1,5-dihydroxynaphtalène, du 2,7-dihydroxynaphtalène, du 1-naphtol, du 2-amino-4-hydroxyéthylaminoanisole et/ou leurs sels physiologiquement acceptables ainsi que leurs mélanges.

9. Procédé selon l'une des revendications précédentes, **caractérisé en ce que** l'agent cosmétique (M2) contient l'au moins un précurseur de colorant par oxydation du type coupleur (M2-2) en une quantité totale allant de 0,0001 à 6,0 % en poids, de préférence de 0,001 à 5,5 % en poids préférablement de 0,002 à 4,5 % en poids, en particulier de 0,005 à 2,5 % en poids, par rapport au poids total de l'agent cosmétique (M2).

10. Procédé selon l'une des revendications précédentes, **caractérisé en ce que** l'agent cosmétique (M2) présente un pH de 5,0 à 12, de préférence de 5,0 à 11, préférablement de 5,5 à 10, en particulier de 6,0 à 9,0.

11. Procédé selon l'une des revendications précédentes, **caractérisé en ce que** l'agent cosmétique (M2) contient au moins un agent oxydant (M2-3) du groupe constitué par des persulfates, des peroxodisulfates, des chlorites, des hypochlorites, du peroxyde d'hydrogène ainsi que ses produits de dépôt solides sur l'urée, la mélamine, la polyvinylpyrrolidone et le borate de sodium, de préférence du peroxyde d'hydrogène, en une quantité totale allant de 0,1 à 6 % en poids, de préférence de 0,3 à 4 % en poids, en particulier de 0,5 à 3 % en poids, par rapport au poids total de l'agent cosmétique (M2).

12. Procédé selon l'une des revendications précédentes, **caractérisé en ce que** le rapport quantitatif entre la quantité totale de l'agent oxydant (M1-1) dans l'agent cosmétique (M1) et la quantité totale de l'agent oxydant (M2-3) dans l'agent cosmétique (M2) présente une valeur (M1-1)/(M2-3) de 16:1 à 1:1, de préférence de 8:1 à 1:1, préférablement de 6:1 à 1:1, de manière encore davantage préférée de 5:1 à 1:1, en particulier de 4:1 à 1:1.
